# EUROPEAN PATENT APPLICATION

(11) **EP 1 852 122 A1**
(43) Date of publication of application: **07.11.2007**
(21) Application number: 07106397.8
(22) Date of filing: 18.04.2007
(51) Int. Cl.: A61K 35/74, A61P 1/02, A61K 8/99, A61Q 11/00

(54) **Dental and gingival health compositions containing reactivatable, dehydrated, eubiotic micro-organisms**

(30) Priority: 02.05.2006 IT MI20060860
(71) Applicant: Truffini & Regge' Farmaceutici SRL, 20134 Milano (IT)
(72) Inventor: Baudo, Giuseppe, I-20137, Milano (IT)
(74) Representative: Pistolesi, Roberto

(57) **Abstract**

The present invention relates to compositions containing one or more reactivatable, dehydrated, live micro-organisms.

In particular, the compositions contain *Enterococcus faecium, Saccharomyces boulardii* and/or mixtures thereof, in combination with pharmaceutically acceptable excipients.

The present invention also relates to the use of the dehydrated live micro-organisms, in particular *Enterococcus faecium, Saccharomyces boulardii* and/or mixtures thereof, for the treatment of diseases affecting the gums and/or the tooth-support tissues.

## Description

Diseases of the gums and of the tooth-support tissues, which are better known as gingivitis or periodontitis, are amongst those that occur most frequently on a world-wide scale involving, it is estimated, as many as 70% of the population in one form or another and 600-900 million people in the form of severe periodontal disease.

Periodontitis constitutes a potential risk factor for other more serious pathologies such as cerebral vascular incidents (risk doubled), chronic respiratory pathologies (from 2-fold to 5-fold increase in risk), coronary pathologies (risk doubled), complications in pregnancy (from 4-fold to 7-fold increase in risk), diabetes (from 2-fold to 4-fold increase in risk). Periodontal diseases are chronic bacterial infections which affect the four tissues that support the teeth, that is, the two soft tissues (gums and ligaments) and the two hard tissues (cement and alveolar bone).

Dental plaque, that is, the bacterial film which covers the teeth, is not *per se* the cause which triggers periodontitis infection and inflammation but, if it is neglected and not removed, it may harden into a calcareous deposit which is known as tartar and which constitutes the medium in which much more virulent bacteria can proliferate; in the developed form of periodontitis these bacteria may lead to loss of the tooth.

Gingivitis constitutes the most benign form of periodontal disease; the gums are reddened and bleed easily and the accumulation of plaque adds irritation to the other problems. Gingivitis is reversible and, in the current state of the art, the conventional treatment consists of good oral hygiene.

Periodontitis is a developed state of periodontal disease which is characterized by inflammation and progressive destruction of the tooth-attachment system, that is, of the four support tissues. The formation of gum pockets of greater or lesser depth between the gum and the tooth root is characteristic of this stage.

Conventional treatments consist of the use of antibiotics, disinfectants, antiseptics and/or antimicrobial agents such as, for example, chlorhexidine.

However, these treatments are not resolutive and have various side effects (anaphylactic reactions, cross-resistances, darkening of the teeth, etc...).

It has now surprisingly been found that live biological agents (micro-organisms) can be used successfully in the treatment of tooth and gum diseases, in particular for the treatment of diseases affecting the tooth-support tissues.

The live micro-organisms act by combating the virulent pathogenic bacterial flora, re-establishing the equilibrium of the affected tissues.

The virulent pathogens, which are represented substantially by anaerobic and gram-negative organisms, produce enzymes and toxins which can destroy the periodontal tissues and inhibit the immune system. Some of the most common virulent pathogens are, for example, *Porphyromonas gengivalis, Prevotella intermedia, Actinobacillus actinomycetemcomitans, Bacteroides forsythus, Eikenella corrodens,* parasites (amoebae and trichomonas), and spirochetes.

The number of beneficial gram-positive bacteria in the mouth decreases greatly as the disease develops; that is, in healthy periodontia, 75% of beneficial gram-positive bacteria are present, in gingivitis, this falls to 45% and, finally, in periodontitis it falls to 25%. The equilibrium (eubiosis) of the mouth system is thus broken down in favour of the virulent bacteria with consequent disequilibrium (dysbiosis).

The live micro-organisms according to the present invention are preferably selected from *Enterococcus faecium, Saccharomyces boulardii* and/or mixtures thereof. The *Enterococcus faecium* according to the present invention is a live micro-organism in dehydrated form that can be reactivated easily in the conditions of use, which are species-specific to man, and that can form permanent colonies on the mucosa of the oral site since it is accepted by our immune system, can effectively combat the other harmful or pathogenic bacteria, and is eubiotic as well as probiotic with regard to the well-being of the human organism.

The *Saccharomyces boulardii* according to the present invention is a micromycete in reactivatable, dehydrated form and is considered a high-profile bio-therapeutic agent that is compatible with man and, unlike the other yeasts, also capable of passing through the gastricacid barrier (pH 1-2) unharmed, and that can effectively combat the other harmful and pathogenic bacteria and mycetes which are present at the site where it is applied.

According to the present invention, the micro-organisms are in a dehydrated form with a moisture content usually of from 3 to 5%. The dehydration is normally performed by known processes such as, for example, the lyophilization process. The micro-organisms may also be protected for the purposes of longer preservation by means of a microencapsulation process.

The dehydrated micro-organisms then have the characteristic that they revive when they are returned to an ecological site which is moist and congenial to them, for example, the intestinal mucosa or that of the oral cavity, where they colonize in species-specific and site-specific form, restoring the equilibrium (eubiosis) between the various resident (autochthonous) or transient (allochthonous) bacterial flora species. This equilibrium usually leads to biological control, by competition, of the harmful bacterial species and to remission of the symptoms that are due to the various pathogens which are thus reduced numerically to below the level at which they are harmful, or are completely eliminated.

A subject of the present invention is therefore compositions in pharmaceutical forms containing one or more dehydrated, live, probiotic micro-organism preferably selected from *Enterococcus faecium, Saccharomyces boulardii* and/or mixtures thereof, in combination with pharmaceutically acceptable excipients.

Acceptable pharmaceutical forms are tablets, operculate capsules, micro-capsules or pearls, powders in sachets or other containers, single-dose bottles, chewing gums, pastes or gels, solutions, emulsions in any format, sprays, liquid mouth-washes or any technologically-available format. The compositions may be classified as prescription or over-the-counter pharmaceutical preparations, medical apparatus or medical-surgical requisites, cosmetics, food supplements, foods, or other categories that are established or may be established by law.

The reactivatable, eubiotic micro-organisms are contained in the compositions according to the present invention in a quantity preferably of between 0.3x10⁹ CFU/dose and 3x10⁹ CFU/dose. According to a preferred embodiment, the compositions of the invention may also contain one or more additional active ingredients which act synergically with the live micro-organisms. Additional active ingredients suitable for the present invention may be selected, for example, from xylitol, immunostimulating polysaccharides (e.g. 1,3-β-glucan), etc.

The compositions according to the present invention are useful in the dentistry field and in the treatment of the oral cavity; in particular, they are useful for the treatment of diseases of the oral mucosa, of the gums, and of the tooth-support tissues, and for re-establishing eubiosis in gingivitis or periodontitis.

In addition, the compositions are useful for treatments to reduce halitosis due to the fermentation of proteinaceous food residues by proteolytic bacteria.

The compositions of the invention may also be swallowed after oral application rather than expelled with saliva since they are compatible and, in particular, also useful as probiotics in various sites of the small and/or large intestine.

According to a preferred embodiment of the invention, the above-mentioned compositions are applied to the mucosa of the oral cavity by means of a normal toothbrush by pressing against the visible bases (necks) of the teeth to promote penetration of the composition of the invention into the gum pockets. Bleeding and many of the other symptoms of periodontal diseases (gingivitis or periodontitis) can thus be reduced.

The following examples are intended to illustrate the present invention further without in any way limiting its subject.

### EXAMPLES

### Example 1

Edible gelatine capsule type 0 containing *Enterococcus faecium,* and *Saccharomyces boulardii* in pre-measured, single-dose form. The edible gelatine capsule contained, by way of example, 1-2 thousand million colony-forming units (CFU) of each of the two probiotic micro-organisms in combination with xylitol and pharmaceutically acceptable excipients.

The capsule was opened manually by pulling at the two ends; it was then applied to the neck of the tooth and gum by rubbing with the aid of a normal moist brush onto which the contents of the capsule were poured. Rinsing was not necessary and the contents of the capsule were left to act for several hours, any excess saliva that formed optionally being swallowed or eliminated. The treatment was repeated until it was no longer necessary.

### Example 2

A hermetically-sealed or hermetically-resealable dispenser containing powder as in Example 1. A brush was used for application as in preceding Example 1.

### Example 3

Bottles with breakable container stoppers (two-phase solid-liquid) for the preparation, as required, of mouth-wash liquids or gels ready for topical use. This system also permits a long life of the live biological product *(Enterococcus faecium, Saccharomyces boulardii* and/or mixture thereof, as in Example 1) which is contained in the stopper. This system keeps the biologically active part of the composition that is contained in the hermetically-sealed stopper away from moisture, light, heat and oxygen prior to preparation, as required, by mixing of the contents of the stopper and of the bottle followed by immediate use of the composition of the invention.

## Claims

1. A composition containing *Enterococcus faecium, Saccharomyces boulardii* and/or a mixture thereof in combination with pharmaceutically acceptable excipients.

2. A composition according to Claim 1 in which the *Enterococcus faecium, Saccharomyces boulardii* and/or mixture thereof is present in reactivatable, dehydrated form.

3. A composition according to Claim 1 further containing one or more additional active ingredients, preferably selected from xylitol, immunostimulating polysaccharides (e.g. 1,3-β-glucan), etc.

4. A composition according to any one of the preceding claims in which the *Enterococcus faecium, Saccharomyces boulardii* and/or mixture thereof is present in a quantity which varies from 0.3x10⁹ CFU/dose to 3x10⁹ CFU/dose.

5. A composition according to any one of the preceding claims in which the *Enterococcus faecium, Saccharomyces boulardii* and/or mixture thereof is present in a quantity which varies from 1x10⁹ CFU/dose to 2x10⁹ CFU/dose (reduced preferred range).

6. A composition according to any one of the preceding claims in powder, tablet, capsule, microcapsule pearl, dental chewing-gum, paste, cream or gel form.

7. A composition according to any one of the preceding claims in solution, emulsion or mouth-wash form.

8. A composition according to any one of the preceding claims in the form of a bottle with a breakable container stopper (two-phase bottle), a hermetically-sealed or hermetically-resealable dispenser and/or a sachet.

9. Use of live probiotic micro-organisms for the preparation of a compound for the treatment of diseases of the oral cavity, preferably of the gums and/or of the tooth-support tissues.

10. Use according to Claim 9 in which the micro-organisms are in reactivatable, dehydrated form and are protected by microencapsulation of the micro-organisms.

11. Use according to any one of the preceding claims in which the micro-organisms are selected from *Enterococcus faecium, Saccharomyces boulardii* and/or a mixture thereof.

12. Use according to any one of the preceding claims in which the micro-organisms are contained in a quantity which preferably varies from 0.3x10⁹ CFU/dose to 3x10⁹ CFU/dose.

13. Use according to any one of the preceding claims in which the compound is for the treatment of gingivitis or periodontitis.

14. Use according to any one of the preceding claims in which the compound is for the treatment of halitosis.
